(19)

**Europäisches Patentamt**

**European Patent Office**

**Office européen des brevets**

(11) **EP 4 220 161 A1**

(12) **EUROPEAN PATENT APPLICATION**

(43) Date of publication:
02.08.2023 Bulletin 2023/31

(51) International Patent Classification (IPC):
*G01N 33/53* (2006.01)   *G01N 33/543* (2006.01)
*G01N 15/14* (2006.01)

(21) Application number: 22153689.9

(22) Date of filing: 27.01.2022

(52) Cooperative Patent Classification (CPC):
G01N 33/54313; G01N 15/1459; G01N 33/5306

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Bundesrepublik Deutschland,
vertreten durch den
Bundesminister für Wirtschaft und Energie
12205 Berlin (DE)**

(72) Inventors:
• **CARL, Dr., Peter**
**10961 Berlin (DE)**
• **SCHWAAR, Dr., Timm**
**12437 Berlin (DE)**
• **GUNDERMANN, Nick**
**12437 Berlin (DE)**

(74) Representative: **Zimmermann & Partner
Patentanwälte mbB
Postfach 330 920
80069 München (DE)**

(54) **A BUFFER SYSTEM FOR PARTICLE-BASED MULTIPLEXED IMMUNOASSAYS**

(57)     A buffer system for detecting an analyte using a suspension array fluorescence immunoassay (SAFIA) comprising: (a) an extract dilution buffer for diluting an extract of a food or a feed sample to be analysed in the SAFIA, the extract dilution buffer comprising: a first detergent having a high hydrophilic-lipophilic balance (HLB), a second detergent having a low HLB, and a polyanionic species, wherein the high HLB is selected from a range of HLB-values of 8 through 13, and the low HLB is selected from the range of HLB-values of 14 through 20; (b) stopping buffer for stabilizing a read-out signal of the SAFIA by preventing unbound antibodies form reacting with a corresponding epitope, the stopping buffer comprising: the analyte, a protein crosslinker, MeOH and SDS, wherein the protein crosslinker is selected from formaldehyde or a linear and/or branched alkane or aromatic dialdehyde selected from: glyoxal, succinic aldehyde, glutaraldehyde, adipaldehyde, benzene dicarbaldehyde, and genipin; and (c) a secondary antibody dilution buffer for diluting a secondary antibody comprising the extract dilution buffer and a polyglycol ether of a fatty acid, wherein the secondary antibody is directed towards a primary antibody and comprises a luminescence label, and wherein the first antibody is directed towards the analyte and/or a hapten fixed at a particle's surface.

Fig. 4

EP 4 220 161 A1

Processed by Luminess, 75001 PARIS (FR)

**Description**

TECHNICAL BACKGROUND

The present invention relates to chemical analysis of food, feed, and environmental samples such as soil- and water samples. More precisely, it relates to the field of immunoassays, particularly to the suspension array fluorescence immunoassay (SAFIA) technology, described elsewhere [1].

The SAFIA technology comprises a particle-based immunoassay, more precisely, a combination of suspension array technology (SAT) and fluorescence immunoassay (FIA). SAT uses encoded microparticles for a multiplexed detection and quantification of different analytes in a sample. For the measurement encoded microparticles are used. Encoding here refers to unique features of individual particles, e.g., their size or shape, which can be discriminated via the measurement of light scattering at different angles or via microscopical techniques. Encoding may also be achieved by luminescence properties, such as different luminescence intensities in a distinct wavelength-range (intensity coding) or luminescence in different wavelength-ranges (spectral coding) or, e.g., different luminescence lifetimes. In practice, coding can be achieved by introducing organic dyes or luminescent particles (i.e., quantum dots, carbons dots, etc.) in or on the particle in different concentrations. Each individual coded particle encodes a known analyte that is measured with this particular type of particle. For measurement of multiple analytes different encoded particles are used in a mixture, thereby achieving multiplexing analysis.

For SAFIA, analogues of analytes, i.e., haptens, are immobilized on the surface of carrier particles. The immunoassay-part of the SAFIA utilizes specific antibodies (abs), that can bind the hapten-structures on the particles as well as the analyte itself. If mixed with a sample that contains the analyte, a competitive reaction takes place between the surface-immobilized hapten and the free analyte in solution (indirect competitive immunoassay). The amount of particle-bound antibody (ab), i.e. the later signalling unit, is anti-proportional to the analyte concentration in solution. The surface bound antibody is then labelled with a secondary antibody, which carries a luminescent label. Next, the particles are analysed using a flow-cytometer, where they are hydrodynamically separated and focused before passing the excitation light sources. Subsequently, the lasers excite present luminophores, i.e., both the luminescent label of the antibody and the luminophore from the particle-code. Luminescence and scattering properties of the particles are then detected with multiple detectors. As the luminescence measurement is correlated to light scattering, bound antibodies on the particles and free antibodies can be discriminated. Therefore, in principle, no washing steps are needed during the procedure which makes SAFIA a fast, easy, and economical method.

To enable a wash-free method, we have demonstrated that favourably a stopping solution consisting of a cross-linker and a denaturation is utilized. This guarantees that the signal of the immunoassay will not change during the read-out, as it would be the case in a non-stopped SAFIA, since the antibody-antigen reaction is not in an equilibrium state.

The later analysis is done by so-called gating of the individual signals collected from the particles with the flow-cytometer while using luminescence based sorting. Setting a gate with distinct intensity values on a specific detector enables downstream analysis: only the signals within the range defined by the gate are used for the consequent evaluation of the immunoassay. Therefore, different encoded particles can be distinguished from each other so that the population of particles comprising one specific code can be evaluated. For quantification of the analyte the median signal of the luminescence intensity of the secondary antibody is correlated with the concentration of the analyte. Therefore, a calibration curve can be used: the luminescence intensity of the calibration measurement is plotted against the concentration of the analyte. For curve-fitting a linear, quadratic or, e.g., a four-parameter calibration model can be applied. The concentration of different analytes in an unknown sample can then be accurately estimated by measuring the luminescence-intensity and applying the obtained calibration model.

STATE OF THE ART

This underlying technique has been applied to measurement of different analytes in a variety of sample materials. However, the described assays require washing steps to circumvent either signal-prolongation during the read-out or the undesired interference of substances, that are not the analyte, from the sample-materials (i.e., matrix).

Currently, we have developed a technique that allows the assay to be executed in a wash-free manner, utilizing polystyrene-core silica-shell particles [1]. The derived SAFIA had three main key-features:

1. The surface of the particles was modified with two functional groups: a group for the coupling of the hapten (Amino-, Carboxy-, Thio-, or other) and a functional group to prevent non-specific binding (PEG, etc.), thus enabling specific-binding of the primary antibodies.

2. The hapten was coupled directly to the surface of the particles. This was done by activation of carboxyl-moieties of the respective hapten-molecule by dicyclohexylcarbodiimide (DCC)/N-hydroxy succinimide (NHS)-chemistry via formation of an amide bond with the amino groups located on the surface of the particles.

3. A stopping solution was utilized, which addressed the issue of signal prolongation during read-out of the assay in the flow-cytometer. The stopping solution contained a crosslinker (e.g. formaldehyde) and an organic solvent (methanol) as well as a surfactant to denature antibodies in solution. This prevented antibodies from binding to the surface during the read-out in the cytometer. Moreover, formaldehyde crosslinked the antibodies bound to the particles, preventing their loss during the read-out. In total, a stable signal over the course of > 2 h was achieved.

DRAWBACKS OF PREVIOUS SOLUTIONS

However, the current state of the art has various drawbacks. From the state of art, it was demonstrated that only aqueous samples could be quantitatively analysed with the current technique. A maize sample extract gave only semi-quantitative results. The overall accuracy of the assay was poor: the recovery rate spanned from 48-54% for ochratoxin A (OTA) and from 53-73% for zearalenone (ZEN) [1]. The ideal value would be in the range of 70-110% for OTA and ZEN according to the EU-Legislation 519/2014. This indicates that quantification of mycotoxins in food and feed samples would be insufficient with this method.

Furthermore, when we tried to analyse food samples with a high protein and starch content, such as samples of grain (wheat, rye, spelt, buckwheat, etc.) or samples with high fat content (almond, hazelnut), or other "difficult" samples (i.e., spices, like pepper) we found that the accuracy was so poor that even a qualitative result was not feasible: all samples were highly overestimated.

The main reason for this overestimation was the suppression of the signal due to matrix-effects. In contrast to aqueous samples, food and feed samples may require extraction with an organic solvent or a mixture of solvents to dissolve the analytes for downstream analysis. Which means that also undesired sample components become extracted as well. In the SAFIA analysis these matrix-components such as proteins and fats bind to the surface of the particles. This results in less or no antibodies bound to the particles, as compared to a standard solution used for calibration without the matrix components. As the signal-to-concentration relationship within SAFIA is governed by indirect proportionality, this leads to the overestimation of the analyte-concentration in the samples. The SAFIA in this manner was not feasible.

Other assays used washing steps to separate matrix-components from assay components during analysis. However, washing cycles are cumbersome in execution and increase the time-to-results and costs per assay. Moreover, they require special equipment, such as filter plates and vacuum manifolds or magnetic washers (if magnetic particles are used). Washing of a particle-based immunoassay requires highly specialized skills for technical personnel, as there are the risks of particle-loss and cross-contamination.

Therefore, a wash-free assay, where the reagents need only to be brought in a mix prior to read-out, is highly desirable. Furthermore, even with washing steps significant matrix-effects might take place.

Therefore, up to the present time, there is no technical solution to achieve a wash-free measurement of samples extracted from food, feed, and/or environmental samples, which was the goal of the underlying invention.

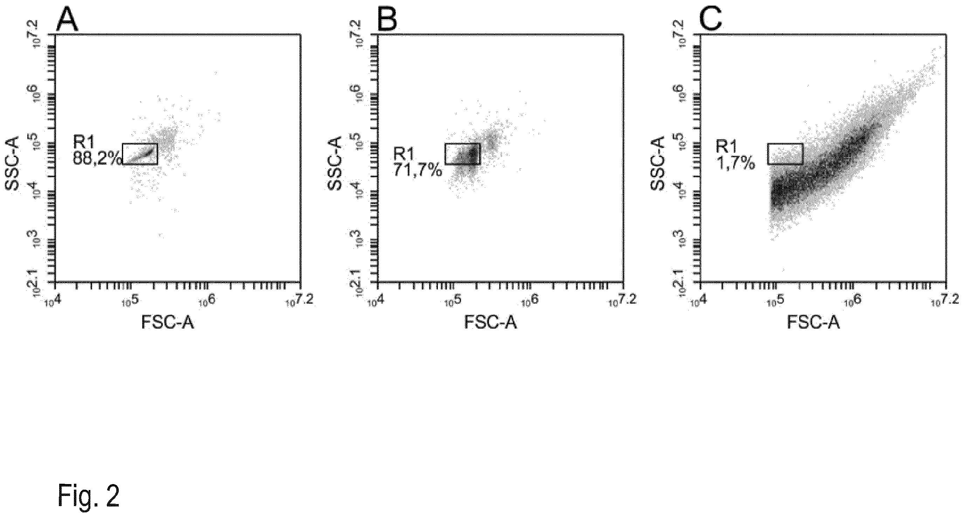

As immunoassays can only operate in aqueous buffers, organic extracts must be diluted in aqueous media prior analysis with an immunoassay. However, when diluting methanolic extracts of, e.g., cereal or nut-products, it was observed that samples become unstable: they form precipitates over time, which are harmful for cytometric read-out, as flow channels with a small diameter get clogged. Also, the assay microparticles adsorb on these precipitates and cease to work properly. Every particle in the sample results in a measurement-trigger. The counted events during read-out cause a flood of unnecessary data slowing down the data analysis. Furthermore, the cytometer does not count enough assay particles, especially when the measurement-stop settings are set to a distinct counting of assay particles in a gate (see Figure 2).

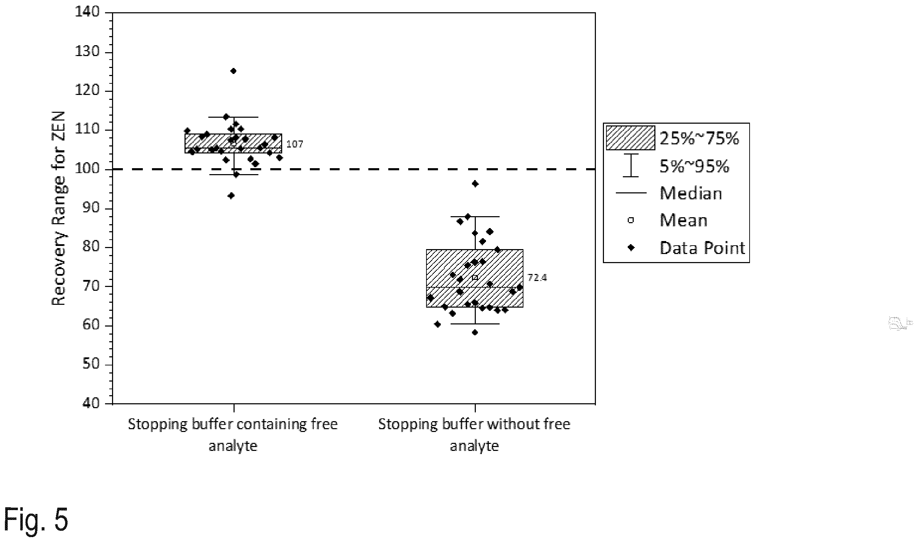

An additional drawback of the previous solution was the insufficient stopping of the assay. As some antibodies are more stable in organic solvents than others, they do not become denatured by the used stopping solution. This results in a slight signal-increase during the read-out of the microtiter plate in the flow-cytometer leading to a lower precision and total accuracy of the assay. For example, the assay for ZEN showed a signal prolongation during the assay, leading to a decreased accuracy (see Figure 5). On further, formaldehyde tends to polymerize, which produces a signal background. Therefore, the same drawbacks apply here in the same manner as for unstable samples.

The challenge, therefore, was to find an aqueous buffer system, that a) stabilizes the sample during flow cytometric read-out and b) prevents matrix-components from interfering with the SAFIA-particles and other assay components. With a suitable buffer system, the accuracy (precision and trueness) of the assay will be improved.

The challenge of stabilization can be addressed using a detergent or surfactant, that would solubilize such components, which are only solvable in organic solvents, e. g., fats and proteins. However, detergents are also known to denaturize antibodies, which would aggravate the accuracy of the assay. Furthermore, detergents can also lead to a decreased luminophore intensity, which decreases the assay sensitivity. For instance, the fluorescence of a fluorochrome can be quenched in this manner. Therefore, a solution is needed either to prevent the signal from decreasing or/and to increase the signal of the immunoassay, e. g., by using additives. In general, a buffer system is required that promotes high signal intensities.

To solve the problem of non-specific binding, a solution must be found that does not interfere with the immunoassay itself, but only with the respective matrix components. In particular, the interaction between positively charged proteins (basic side residues) and the negatively charged particles (deprotonated silanol residues at high pH) has a major impact on the accuracy of the assay: when these proteins bind to the particles, antibodies are hindered from binding to the hapten, which leads to a decreased or completely lost signal. Usually, surfaces involved in immunoassays (e. g., the surface of polystyrene microtiter plates in ELISA) are blocked using large amounts of proteins. Common is the use of bovine serum albumin (BSA) or casein as blocking agent. These proteins bind to the free binding sites on the surfaces (e.g., polystyrene sites, where no protein is adsorbed) and prevent assay components from binding, which would lead to a false-positive signal.

The binding of the blocking-proteins is based on hydrophobic and/or ionic interactions between the surfaces. For instance, proteins undergo hydrophobic interactions with the hydrophobic polystyrene or ionic interactions with functional groups that are created via plasma-oxidation of the polystyrene. Therefore, blocking is a common solution to address this issue. However, for SAFIA blocking of the particles is not applicable: When blocked with BSA, we found that the signal-intensity drops significantly with an increasing amount of BSA added to the particles. Therefore, a solution must be found that blocks the interference in solution and, at the same time, is compatible with the demands for a stable sample.

The challenge of improving the stopping solution was to find a way from preventing the unbound antibodies from binding to the particles during the read-out of the plate. Replacing formaldehyde by glutaraldehyde prevented the problem of unwanted polymerization and consequent aggregate formation. Moreover, compared to formaldehyde, glutaraldehyde offers the advantages of decreased toxicity and increased stability.

However, any attempt to increase the effectiveness of denaturization were unsuccessful: using higher concentrations of methanol and/or SDS, addition of known denaturization agents (guanidinium thiocyanate, $Ca^{2+}$-ions) resulted either in a high-level of unwanted polymerization and/or did not prevent signal prolongation. Therefore, a new solution other than denaturization had to be invented.

SUMMARY

The invention involves the use of a buffer system composed for SAFIA analysis of food and feed samples. The first key component is a sample buffer that stabilizes the samples and avoids non-specific binding of matrix components and thus increases the overall accuracy of the assay. The second key component is a buffer which increases the luminescent signal of the fluorochrome labelled secondary antibodies and therefore increases the sensitivity of the assay. The third key component is an optimized stopping buffer that prevents signal prolongation.

According to an embodiment, a buffer system for detecting an analyte using a suspension array fluorescence immunoassay (SAFIA) is suggested. The buffer system comprises:

a) an extract dilution buffer for diluting an extract of a food or a feed sample to be analysed in the SAFIA, wherein the extract dilution buffer comprises:

- a first detergent having a high hydrophilic-lipophilic balance (HLB) ,
- a second detergent having a low HLB, and
- a polyanionic polymeric species,

wherein the high HLB is selected from a range of HLB-values of 8 through 13, and
the low HLB is selected from the range of HLB-values of 14 through 20;

b) a stopping buffer for stabilizing a read-out signal of the SAFIA by preventing unbound antibodies form reacting with a corresponding epitope, wherein the stopping buffer comprises:

- the analyte,
- a protein crosslinker,
- MeOH and
- SDS,

wherein the protein crosslinker is selected from formaldehyde and a linear and/or branched alkane or aromatic dialdehyde (e. g. glyoxal, succinic aldehyde, glutaraldehyde, adipaldehyde, benzene dicarbaldehyde or genipin); and

c) a secondary antibody dilution buffer for diluting a secondary antibody comprising the extract dilution buffer and a polyglycol ether of a fatty acid,

wherein the secondary antibody is directed towards a primary antibody and comprises a luminescence label, and

wherein the first antibody is directed towards the analyte and/or a hapten fixed at a particle's surface.

Advantageously, the suggested buffer system allows for SAFIA analysis of extracts of food, feed, and other agricultural products for various analytes. Using the buffer system, the SAFIA shows a high signal intensity and therefore a high sensitivity, which enables the user to detect harmful substances in various products, according the EU Legislation. The extraction buffer stabilizes the sample for analysis, i. e. particles formation is successfully prevented and significantly reduces matrix-interferences via inhibition of non-specific binding. Advantageously, this improves the assays accuracy (precision and trueness). Also, the prevention of aggregate formation originated form the sample prevents clogging of the reading devices (flow cytometers) and therefore prevents severe damage to these instruments. The proposed stopping solution allows for a stable measurement over the course of several hours and therefore improves the accuracy of the assay. Also, the handling of the assay is improved, as washing steps are eliminated. Advantageously, this shortens time-to result, and allows the assay to be performed by non-skilled personnel, as the reagents simply must be mixed. On further, typical problems with washing of particles-based assays (e. g. particle loss, cross-contamination) are avoided.

According to an embodiment, a concentration of the polyglycol ether of the fatty acid in the secondary antibody dilution buffer is selected from a value of 0.1 - 0.3% (m/m), and preferably comprises 0.2% (m/m).

Advantageously, such concentration values allow for enhancement of the luminescence of dye-labelled secondary abs, therefore increasing the sensitivity of the assay, without denaturing abs. Also, it compensates the small signal depletion caused by ingredients of the extraction mixture (mostly methanol and SDS). Therefore, these substances do not need to be removed from the assay-mixture, enabling a wash-free execution of the assay.

According to an embodiment, the first detergent of the extract dilution buffer having the high HLB is selected from SDS, and a concentration of SDS is selected from a value of 0.05 - 0.15% (m/m), and preferably comprises 0.1% (m/m).

Advantageously, such concentration values allow for solubilization of fats and proteins and prevents aggregate formation, while primary and secondary abs are not denatured and matrix-effects are reduced, which improves the assays accuracy.

According to the same embodiment, the second detergent of the extract dilution buffer having the low HLB is selected from polysorbate 20, wherein a concentration range of polysorbate 20 is selected from a value of 0.15 - 0.3% (m/m), and preferably comprises 0.2% (m/m).

Advantageously, such concentration values allow for solubilization of fats and proteins and prevents aggregate formation, while primary and secondary abs are not denatured and matrix-effects are reduced, which improves the assays accuracy. Thus, the abovementioned advantages are obtained by combining the two detergents and the polyanionic species with the other. With the combination of the two different detergents with the other components and the polyanionic substance proposed according to the invention, the effects described here as advantageous result.

According to an embodiment, the polyanionic species is selected from a polyanionic polymer, wherein pendant groups of the polyanionic polymer are selected from: hydroxyl, carboxyl, sulfonate, sulfate, sulfite, phosphate, and phosphonate groups and their combinations; and a backbone of the polyanionic polymer is selected from: a polyvinyl, a polystyrene, a polyacrylic, and a polyglycoside, wherein the polyanionic polymer comprises: a polystyrene sulfonate, a polyvinyl sulfonate, an alginic acid, a polyacrylamidomethylpropane sulfonic acid, a polyvinylacetic acid, a polyvinyl phosphonic acid, a polyvinyl sulfonic acid, a carboxymethyl cellulose, a carrageenan, a polyaspartic acid, a polyglutamic acid, a polyphosphoric acid, a polysaccharide, a pectin, a polyacrylic acid and a heparin, particularly a sodium salt of the polyacrylic acid, wherein the polyacrylic acid has an average molecular weight of 1800 through 2200 Da, preferably comprising 2100 Da, and wherein a concentration of the sodium salt of the polyacrylic acid is selected from a value of 0.5 - 3% (m/m), preferably comprising 0.1% (m/m).

Advantageously, the indicated polymers are well water soluble. Further, the indicated concentration values are valid also for the other polyanionic substances and allow for minimizing undesired interferences by matrix-components, such as proteins, via blocking of basic sites of proteins, without compromising the antibodies binding sites. Classical immunoassays, like ELISA, use washing steps to eliminate these interferences, while the SAFIA does not need to be washed.

According to an embodiment, the stopping buffer is configured to stabilize a bond between a secondary antibody or a primary antibody and an entity which is specifically recognized by said primary or secondary antibody. Therein said entity typically comprises the primary antibody which has already bound (i.e. recognized) either the corresponding hapten which is fixed at a particle's surface or the corresponding analyte, which is presumably present in the sample extract in a dissolved state. In other words: The stabilization of the bond between primary and secondary antibodies stems from the crosslinker in the proposed buffer system. In particular, a suppression of the otherwise observed signal increase as time progresses is caused by the addition of the analyte to the stop solution. The analytes saturate the free primary antibodies (i.e., those in solution), thus preventing them from binding to the particles, i.e. the buffer system used blocks the binding sites of the primary antibody so that it can no longer bind to the hapten after the assay has been performed.

Advantageously, this allows for a wash-free execution of the assay, with accompanying advantages. On further, the signal is stabilized during the read-out, which takes places over the course of few hours. Signal stability here, leads to improved accuracy.

According to an embodiment, the secondary antibody dilution buffer further comprises laureth-23 in a concentration range of 0.1 - 0.3%.

Advantageously, laureth-23 enhances a luminescence of the luminescence label.

According to an embodiment, an aqueous phosphate buffered saline (PBS) is used as a basis for the buffer system, wherein the PBS typically comprises 100-300 mg/L potassium chloride; 100-300 mg/L sodium or potassium dihydrogen phosphate; 900 -1500 mg/L di-sodium or di-potassium hydrogen phosphate (anhydrous); and 6000-10000 mg/L sodium chloride.

Advantageously, PBS is well established in biochemical assays. It is cheap, commercially available and adapted to nearly all kind of antibodies (IgG and IgM classes) from mouse, rabbit, goat, horse, hen, camel, and other animals. It is however noted that other buffers, e.g. Tris-HCl, borate buffer, acetate-buffer, Good-buffers (HEPES or MES Buffer), citrate buffer, glycine-buffer or buffers, that use a combination of these typical buffer substances, could be used as well as a basis buffer. The selection of a specific buffer system may vary depending on the sample that is analysed or the specific label of the secondary antibody that is used, or depending on the primary or secondary antibodies themselves, which might have different pH-dependent affinities to the target structures (analytes, haptens or primary antibodies).

According to an embodiment, an aqueous TRIS buffered saline (TBS) is used as a solvent for the buffer system, wherein the TBS typically comprises 800-1500 mg/L Tris(hydroxymethyl)aminomethane and 6000-10000 mg/L sodium chloride and optionally 100-300 mg/L potassium chloride.

Advantageously, TRIS buffers are favoured for their buffer capacity.

According to an embodiment, an aqueous borate buffered saline (BBS) is used as a solvent for the buffer system, wherein the BBS typically comprises 1900-12,000 mg/L sodium borate decahydrate (borax) and 6000-10000 mg/L sodium chloride and optionally 100-300 mg/L potassium chloride.

Borate buffers are favoured for their stability and antimicrobial properties.

According to an embodiment, a kit for a SAFIA comprising the buffer system according to any of the previous embodiments is suggested.

Advantageously, said kit ensures a quick and reliable execution of the SAFIA, as all necessary materials are ready-to-use. This enables the user to analyse food, feed, or agricultural products in a quick and reliable manner, according to e. g. EU Legislation.

According to an embodiment, the kit further comprises at least two different lots of core/shell microparticles carrying at their surface different haptens or analytes, at least two primary antibodies, wherein each of said two antibodies is specifically directed to another one of the different haptens or analytes.

Advantageously, at least two different analytes can be detected at a time using the said kit. Advantageously, the provided particles and antibodies are ready-to-use as well, ensuring a fast, reliable, and cheap analysis of food, feed and agricultural products. Advantageously, the detection of multiple analytes at a time shortens time-of-analysis, reduces the costs of the analysis and grand deeper knowledge of the sample composition, e. g. the origin of contaminations in food, feed, and agricultural products.

According to an embodiment, any of the primary antibodies of the kit is directed to another analyte, especially to another mycotoxin, wherein the mycotoxin is selected from: an Ochratoxin A, a Deoxynivalenol, a Zearalenone, a Fumonisin (preferably Fumonisin B1 or Fumonisin B2), an Aflatoxin (preferably Aflatoxin B1, Aflatoxin B2, Aflatoxin G1, Aflatoxin G2 or Aflatoxin M1), an ergot alkaloid (preferably ergocornine/ergocorninine, ergocristine/ergocristinine, ergocryptine/ergocryptinine ($\alpha$- and $\beta$-forms), ergometrine/ergometrinine, ergosine/ergosinine or ergotamine/ergotaminine); a T2 toxin; and a HT2 toxin.

Advantageously these mycotoxins are the most abundant and relevant ones which, according to corresponding legislation, must be monitored.

According to an embodiment, the kit further comprises a secondary antibody which is labelled with a luminophore and directed towards at least one of the primary antibodies. Optionally, the kit may comprise additionally or instead of the labelled secondary antibody another binder molecule carrying a luminescent reporter.

Advantageously, such luminophore markers generate a signal, which is detectable in conventional fluorometric set-ups. In addition, compared to enzymatic immunoassays that use enzyme-catalyzed reactions to generate a readable signal, no signal-generating step is required. Therefore, the use of this embodiment is less expensive and faster compared to similar formats, which is advantageous.

According to an embodiment, using the above described buffer system or the disclosed kit is suggested for determining a concentration of a contaminant in an extract of a sample, wherein the contaminant is selected from a pesticide, an antibiotic, and an allergenic compound, and wherein the sample is selected from a food, a feed, and an agricultural product.

Using the buffer system and the corresponding kit provides the indicated advantages.

According to an embodiment, using the above buffer system and/or using the above kit for determining a concentration of a mycotoxin in an extract of a sample is suggested. Said sample is advantageously selected from a food, a feed, and an agricultural product.

As the concentrations of mycotoxins in this type of samples are regulated by EU Legislation, they must be monitored in order to ensure safety for consumers. Advantageously, the proposed buffer system allows performing a reliable wash-free SAFIA which provides in a short time reproducible results and which does not require highly qualified personnel.

Each embodiment described above may be combined with any other embodiment or embodiments unless clearly indicated to the contrary.

As indicated above, the sample buffer is composed of two detergents that differ significantly in their hydrophilic-lipophilic balance (HLB) values. The HLB can be calculated as stated by Griffin according to equation I

$$HLB = 20 \times \left(1 - \frac{M_L}{M}\right); \qquad (I)$$

where $M_L$ is the molecular weight of the lipophilic part of the surfactant molecule and $M$ is the molar mass of the entire molecule.

The suggested approach towards detecting in a multiplex assay format involves mixing a high HLB detergent and a low HLB detergent to resolve sample compartments without compromising antibody integrity. According to typical embodiments, the mixture of these detergents and especially the concentration of both types of detergents were adjusted to be suitable for both purposes: to stabilize the sample (i.e., to prevent aggregate formation) and to avoid a negative influence on the signal intensity, which might be caused, e.g., by antibody denaturation or luminescence quenching.

We found that a concentration of sodium dodecyl sulphate (SDS, with an HLB of 8.3) in the range of 0.05-0.15% (m/m), preferably 0.1% (m/m), and a concentration of polysorbate 20 (HLB of 16.7) in the range of 0.15-0.3%(m/m), preferably 0.2%(m/m), are stabilizing the samples in such a manner that no particle-aggregates are formed during the read-out. Thus, the sample is stabilized and can be analysed with a flow cytometer set-up.

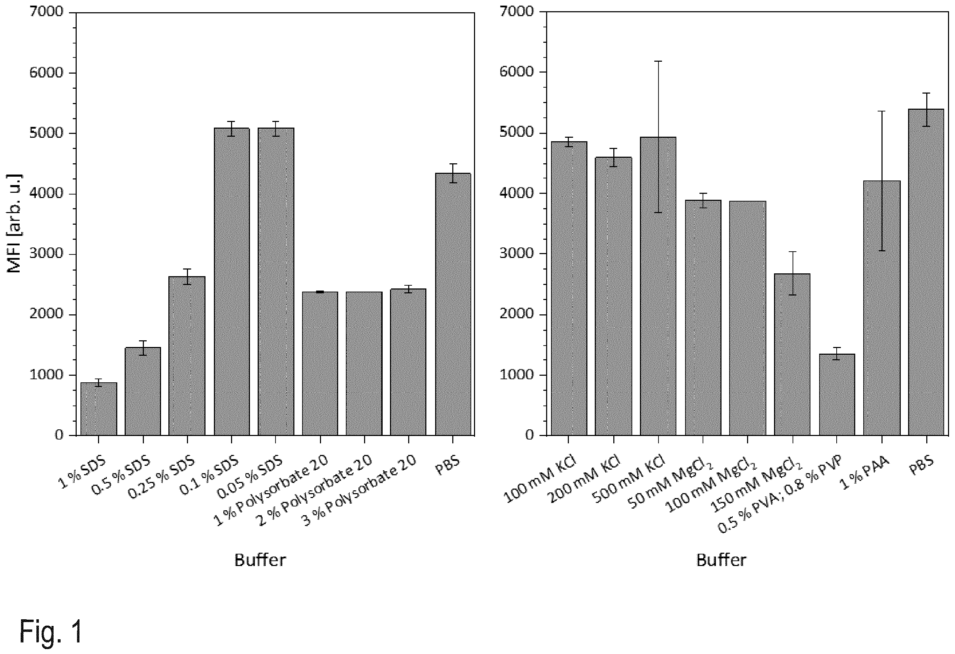

Furthermore, using an anionic detergent and a non-ionic surfactant resulted in a higher antibody stability than if only anionic surfactant or a non-ionic surfactant alone is used. This can be inferred from the decreased signal intensity illustrated by Figure 1. By using SDS as the only detergent in the sample buffer, the samples were not stabilized enough, and particle aggregation was observed. However, SDS at low concentrations was tolerated by the antibodies and the accuracy of the assay was proved to be high.

On the other hand, polysorbate 20 did stabilize the samples, however, the signal-intensity was suppressed, which is a sign for antibody denaturation and/or fluorescence quenching. Therefore, by using both surfactants, a lower level of each surfactant could be used, which resulted in a satisfactory accuracy and sample stability.

To solve the problem of non-specific binding, it is proposed according to the invention to use a polyanionic substance. The polyanionic substance is selected from a polyanionic polymer, wherein the pendant groups of the polymer are selected from: hydroxyl, carboxyl, sulfonate, sulfate, sulfite, phosphate, and phosphonate groups and their combinations. Therein the backbone of the polymer is selected from the group consisting of: polyvinyls, polystyrenes, polyacrylics, and polyglycosides. Examples of suitable polyanionic polymers comprise: polystyrene sulfonate, polyvinyl sulfonate, alginic acid, polyacrylamidomethylpropane sulfonic acid, polyvinylacetic acid, polyvinyl phosphonic acid, polyvinyl sulfonic acid, carboxymethyl cellulose, carrageenan, polyaspartic acid, polyglutamic acid, polyphosphoric acid, polysaccharides, and pectins. Further examples are polyacrylic acid and heparin. As a practical example the sodium salt of polyacrylic acid (with an average mole weight of 2100 Da) was used in a concentration range between 0.5-3% (m/m), with a preferred concentration of 1% (m/m). As the negative charged polyanions bind to positively charged (basic) protein-side residues, the charge of the proteins is neutralized, and they are not able to bind to the negatively charged surfaces anymore. Moreover, by increasing the negative charge of the proteins by both a polyanion and SDS, intermolecular forces become significant enough to repel particles and matrix components from each other. Thereby, they inhibit the non-specific binding of proteins and an increase in the accuracy of the assay follows. Furthermore, the addition of polyacrylic acid also had a stabilizing effect on the samples and even less particle formation was observed in the combination with SDS and polysorbate 20.

As a base for the extract dilution buffer PBS (Potassium chloride: 200 mg/L Potassium dihydrogen phosphate: 200 mg/L di-Sodium hydrogen phosphate anhydrous: 1150 mg/L Sodium chloride: 8000 mg/L) was used. However, all buffers commonly used in biochemistry can be applied as a base as well. Especially the pH of the respective buffer system might be helpful to fine-tune the described effects. The buffer can be prepared directly prior to measurement or as a 10-fold concentrate stock solution, which is then diluted prior sample preparation.

Only the combination of the above three components: the extract dilution buffer, the stopping buffer, and the secondary antibody dilution buffer allowed for a successful and wash-free SAFIA. The samples were stabilized, and the non-specific interactions were significantly reduced. The signal enhancement due to the optimized secondary abs buffer allowed for a more sensitive SAFIA. Also, slight signal prolongation during read-out was sufficiently suppressed. In conclusion, the urge for washing steps was eliminated, resulting in a faster, easier, and cheaper analytical method.

Regarding the issue of signal prolongation during the read-out, the solution was to add free analytes (e.g., mycotoxins, anthropogenic markers etc.) to the stop-solution creating a new stopping buffer. In time-limited immunoassays the antibody-antigen reaction does not reach an equilibrium state. Therefore, antibodies will continue to bind the haptens on particles until an equilibrium is reached, which might take several hours to achieve. The binding of the hapten is slower than the binding of an excess level of analyte in solution, as the rate of the reaction is concentration-depended and diffusion-limited. The concentrations of haptens are usually set to a minimum to reach the so-called affinity limit and the highest sensitivity of an immunoassay. Therefore, when adding a high amount of free analyte after the competitive reaction, the unbound antibodies will bind the free analyte rapidly. In this way, the antibodies are successfully hindered from binding to the haptens on the surface of the particles as the binding sites are blocked after a certain incubation time. Already bound antibodies, however, will not bind the free analyte. Reason for this is, that the dissociation rate of the antibody-antigen complex is very slow and bound antibodies are crosslinked on the surface of the particles via glutaraldehyde. The result is an increased signal stability over the course of time after stopping the competitive reaction of the assay.

The third part of our solution, next to the sample buffer and stopping buffer is a buffer for the secondary antibodies, that increases the luminescence properties. Here, we found that adding polyglycol ethers of fatty acids (narrow-range ethoxylates, NRE) are enhancing the luminescence properties of the secondary antibodies. Especially, polyoxyethylene (23) lauryl ether (laureth-23) in the concentration range of 0.1-0.3%(m/m), preferably 0.2%(m/m) is well suited for this purpose. However, all other polyglycol ethers of fatty acids that have a high HLB-value might be suited as well. By increasing the luminescence properties of the secondary antibodies not only the general sensitivity of the assay is enhanced, but also the slight signal-depletion of the strong surfactants (SDS and polysorbate 20) in the sample buffer is corrected. In addition, we found that glutaraldehyde slightly quenched the luminescence more so than formaldehyde. Use of the proposed buffer for secondary antibodies, however, corrected this issue. Therefore, a combination of these components may favourably be used.

Some aspects of the inventions can be summarized as follows.

1) By choosing an optimal buffer system for particle-based immunoassays, food, and feed samples with complicated matrices (high protein and fat content) can be analysed without the demand for washing steps.
2) A sample buffer that combines a surfactant with high HLB-value and low HLB-value and a poly-anionic species is used to stabilize the sample (i.e., to prevent aggregate formation of sample material) and to reduce non-specific binding to improve the accuracy of the assay.
3) Addition of free analytes to the stopping buffer prevents undesirable signal prolongation.
4) In combination with 2 and 3: a buffer for secondary antibodies is used that enhances the luminescent reporter-signal and therefore increases the sensitivity of the assay. The combination also balances possible signal losses which are caused using a harsher sample/stopping buffers.

BRIEF DESCRIPTION OF DRAWINGS

Figure 1. Optimization of detergent, salt, and additive concentrations.

Figure 2. Examples of scatter plots (FSC vs. SSC) of a stable sample (A), mediocre stable sample (B) and unstable sample (C).

Figure 3. Waterfall-scheme for performance evaluation of the buffers.

Figure 4. Evaluation of the four best buffers with a broad variety of matrix-samples. A) Comparison of blank signal intensities of the sample buffers without food extracts. B) Comparison of intensities of sample buffers with food extracts. C) Signal recovery rates of different buffers with food extracts.

Figure 5. Comparison of recovery rates for ZEN using stopping buffer with and without the free analyte.

Figure 6. Calibration curves of the five plex SAFIA for detection of OTA, DON, ZEN, FB1, and AFB1.

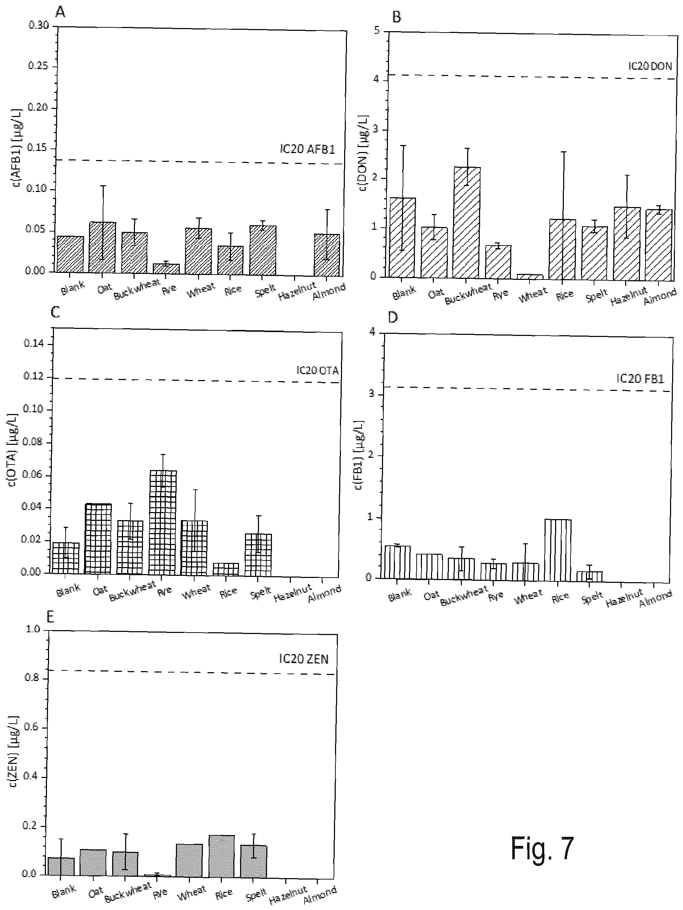

Figure 7. Results from analysis of the 8 different food samples with the suspension array fluorescence immunoassay (SAFIA) and comparison to the IC20-Values (LODs). The LODs are indicated by the dashed lines.

Figure 8. Intra-assay precision (A) and trueness (B) of the SAFIA for AFB1, DON, FB1, OTA and ZEN. The nine spiked samples (pure buffer, buckwheat, spelt, maize, oats, rice, rye, almond and wheat) were analysed in triplicates.

## ADVANTAGES OF THE INVENTIVE SOLUTION

The SAFIA can be executed without washing steps, allowing for the detection of various analytes in a sample in a fast and easy feasible manner. Therefore, time of analysis is shortened, and the assay can be executed by non-trained personnel. The risks and disadvantages associated with particle washing are eliminated: a) there is no further equipment needed for performing the assay; b) laboratory consumables (filters, washing buffers) are spared and c) loss of particles during the washing step is avoided.

Due to the increased signal intensities, caused by the combination of the described buffers, the assay has an increased sensitivity. As the result, the assay can detect lower concentrations of toxins in food and feed samples and therefore advantageously increase the food and feed security.

Complicated matrices like cereal materials (e.g., wheat, barley, rice, spelt, rye), but also nuts (e.g., almonds, hazelnuts, walnuts, etc.) as well as herbal products and spices can be analysed by the SAFIA method, since these samples have been stabilized, and matrix effects have been sufficiently suppressed. Because SAFIA is a fast and easy analytical method, this type of samples can be analysed more rapidly and with a higher accuracy than before.

The advantages of the improved stopping buffer containing free analytes are similar: due to an increased signal stability and the prevention of signal prolongation, the assay accuracy and precision are increased. Moreover, the resulting SAFIA does not depend anymore on denaturization of antibodies to limit the binding time. This means, that even other binders (peptides, enzymes, aptamers, or molecularly imprinted polymers /MIPS) now can be used as analyte recognition element. Suffice to say, this is also the best substitution of washing steps in an assay with all accompanying advantages.

During read-out in the flow cytometer, the formation of aggregates caused by matrix compounds is sufficiently suppressed. In fact, this makes the analysis of these samples feasible, as aggregate formation would have a large influence on the results. The cytometer counts the SAFIA particles in a gate, which triggers the stopping of the measurement. If the SAFIA particles are overshadowed by an overall large number of particles in the sample, there is a risk that not enough SAFIA particles will be counted, required for the robustness of the method. Furthermore, particle formation can lead to aggregation of SAFIA particles and particles originated from the matrix, which would also negatively affect the accuracy of the assay. In addition, formation of large matrix-particles might lead to clogging of the instrument and cause severe malfunctions.

Advantageously, a SAFIA analysis executed with the buffer-system as suggested above will result in a higher accuracy. Further, false-positive and negative results can reliably be avoided.

## PRACTICAL EXAMPLES

### Preparation of Microparticles

Preparation of the particles was done similar to techniques described before [1-3].

Carboxyl-functionalized polystyrene microparticles were encoded using a NIR-emitting BODIPY-dye using the previously described swelling technique in tetrahydrofuran/water mixtures. Then, a silica shell was formed around the particles in an ammonia catalysed Stöber process, using tetraethoxysilane and (3-aminopropyl)triethoxysilane (APTES) as precursors. The surface of the shell was then modified with APTES and 3-[methoxy(polyethyleneoxy)propyl]trimethoxysilane (6-9 PEG units). To the resulting amino moieties on the surfaces, haptens of aflatoxin B1 (AFB1), ochratoxin A (OTA), fumonisin B1 (FB1), deoxynivalenol (DON) and zearalenone (ZEN) were coupled via activation of the carboxylic acid groups using DCC/NHS-chemistry. OTA and FB1 were coupled directly, hapten-derivatives of AFB1, DON and ZEN were prepared according to the known procedure described in the literature [4 - 6]. The coupling was done in a manner that one hapten was attributed to one encoded particle population.

### SAFIA Procedures and Analysis

### Extraction of Food Samples

5 g of the corresponding food samples were weighted into a 50 mL centrifuge tube. Then 25 mL of 70% MeOH in water were added and the sample was shaken in a rotator for 15 min. Solid material was separated via centrifugation and the supernatant was used for further analysis. The extracts were then either spiked to give reference sample materials for accuracy assessment or used in the next step without further processing. For analysis the supernatants were diluted 1:5 in the respective sample buffer and centrifuged again prior analysis to clear turbid samples. The supernatant was then analysed by SAFIA.

SAFIA Analysis

Two different protocols (A and B) were applied for SAFIA analysis. Concentrations of reagents, and applied buffers can be found in Table 1.

Protocol A

50 $\mu$L of the diluted sample or calibrator was applied to a black non-binding microtiter plate, followed by 50 $\mu$L of primary antibody (a mixture of AFB1, OTA, DON, FB1 and ZEN specific antibodies). The mixture was incubated for 5 min on an orbital shaker and then 20 $\mu$L of a mixture of the SAFIA particles were added. The microtiter plate was agitated again for 5 min, then 100 $\mu$L of secondary Alexa488 anti-mouse IgG(H+L) antibodies were added. The plate was incubated for another 20 min. Finally, 100 $\mu$L of the PBS-based stopping solution containing 2% glutaraldehyde as a crosslinker, 0.1% SDS and 10% MeOH were added. The plate was eventually incubated for 5 min and the read-out with an Accuri C6 plus flow cytometer.

Protocol B

Protocol B was an optimized version and is very similar with the protocol A, however, only half of each volume of reagents and samples was used to spare reagents. In addition, the sample, primary antibodies, particles, and secondary antibodies were mixed immediately one after another and incubated for 20 min. We found that there was no significant difference in assay performance (sensitivity, accuracy, signal intensities) comparing both protocols. However, Protocol B allowed for a faster analysis, as incubation times between the steps are avoided.

Table 1. Concentrations and dilution buffer of the SAFIA reagents.

| Dilution of antibodies | | |
|---|---|---|
| Antibody, clone number | Dilution factor | Buffer |
| DON (Clone 2) | 1:10,000 | TRIS[1] or Ab-Stabilizer[2] |
| OTA (CPA9911) | 1:10,000 | TRIS[1] or Ab-Stabilizer[2] |
| ZEN (ZEA-001) | 1:10,000 | TRIS[1] or Ab-Stabilizer[2] |
| FB1 (2A2) | 1:10,000 | TRIS[1] or Ab-Stabilizer[2] |
| AFB1 (5B3) | 1:5,000 | |
| Alexa488 anti-mouse IgG(H+L) | 1:1,000 | TRIS[1] |
| | | |
| Dilution of particles | | |
| Particle code | Final concentration | Buffer |
| Code 1 FB1 | 1% | TRIS[1] |
| Code 2 ZEN | 1% | TRIS[1] |
| Code 3 DON | 1% | TRIS[1] |
| Code 4 OTA | 1% | TRIS[1] |
| [1]10 mM (tris(hydroxymethyl)aminomethan), 150 mM NaCl, 0.05% ProClin 300, [2] TRIS-based, purchased from CANDOR Bioscience GmbH | | |

Optimization of the Sample Buffer and Buffer of the Secondary Antibodies

For optimization of the buffer, we iteratively tested 7 different detergents (SDS, polysorbate 20, sodium deoxycholate, laureth-4, laureth-23, ceteth-2, ceteth-20, and octxinol-9) in combination with 3 different salt additives (KCl, $CaCl_2$, $MgCl_2$), a complexation agent ($Na_2EDTA$) and three polymeric additives (sodium salt of polyacrylic acid (PAA), polyvinylpyrrolidone (PVP), and polyvinylalcohol (PVA)). Each compound was tested in different concentrations as well.

Results

Optimization of the sample buffer

A single-plex SAFIA for the determination of OTA was chosen in the optimization of the sample buffer, as the matrix-effects on this assay were most severe.

First, an optimal concentration for the detergents SDS and polysorbate 20 was determined. SDS was tested in a concentration range between 0.05% and 1% and polysorbate 20 was tested independently in a concentration range between 1-3%. The signal intensities were compared to PBS (Figure 1). A concentration of SDS higher than 0.1% in a similar way as a concentration of polysorbate 20 higher than 1% lead to a decreased signal intensity compared to PBS. Therefore, in the following experiments the concentration was set to 0.1% of SDS and 0.2% of polysorbate 20. In addition, different additives and salts were tested. We found that an additional amount of KCl (100-500 mM), $MgCl_2$ (50-100 mM) or PAA was suitable, as the signal intensities were higher or at least 80% of the ones obtained in PBS.

Afterwards, the sample stability in PBS with one additional component as sample buffer was evaluated. Thereby, the sample extracts of wheat and rye were diluted in the buffer and a SAFIA was conducted. The evaluation was done by visual inspection and comparison of the counted light-scattering events in the flow cytometer. In stable samples, in the plot of the forward scatter (FSC) vs. side scatter (SSC), only one population of the SAFIA particles (with a minor population of aggregated twin-particles) can be observed. In contrast to this, an unstable sample is indicative through a broad distribution of an "event-cloud". Mediocre stable samples had a broader distribution of particles than the stable sample, but with far less events counted than in the unstable sample. It is important to emphasize, that the sample instability occurred even after extra filtration and centrifugation cycles and cannot simply be avoided by these separation methods. An example of a stable, a mediocre stable sample and unstable sample is given in Figure 2.

We found that the buffers with additon of $CaCl_2$ (50-150 mM) or 0.05-0.25% SDS as only one additional component to PBS resulted in an unstable sample. All other additives (KCl, polysorbate 20, PAA, $MgCl_2$) gave mostly stable samples, with some minor mediocrestable samples. Hence, $CaCl_2$ was exlcuded from further considerations.

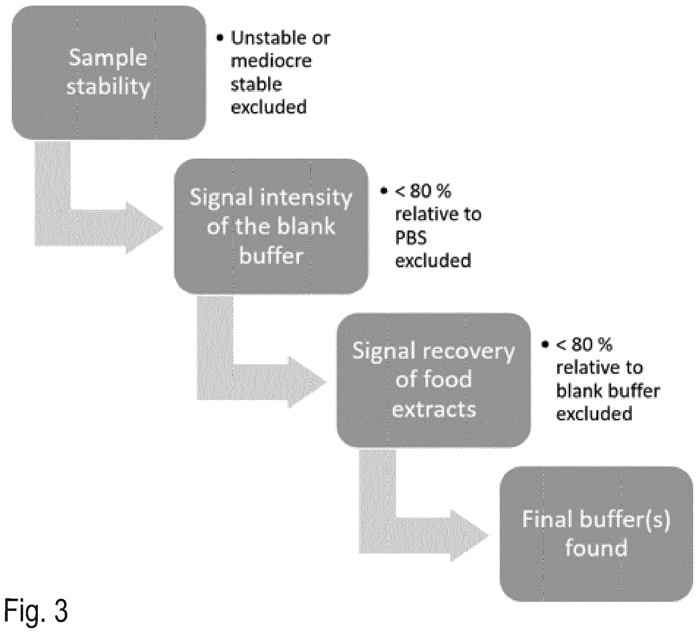

In the next step, sample buffers with more than one of the additional components were tested. The results can be found in Table 2. The perfromance evaluation of the buffers was done by the following waterfall-scheme, see Figure 3.

First, buffers that did not completely stabilize the sample were excluded. Then, only buffers with a relative signal intensity of >80% in comparison with PBS were included in further considerations. Eventually, the signal recovery of wheat and rye extracts (i. e., the signal intensity of a sample in a buffer divided by the signal intensity of a buffer without the sample extract) was used as final criterium. A buffer was considered if the signal recovery of the samples was higher than 80% (ideal: 100%). Here, the buffer with 0.1% SDS, 0.2% polysorbate 20 and 1% PAA showed the best performance (signal recovery $\geq$90%).

Table 2. Evaluation of possible sample buffers with multiple components. Suitable buffers are highlighted in grey.

| Components (SDS: 0.1%, polysorbate 20: 0.2%, KCl: 100mM, MgCl2: 100 mM, PAA: 1%) | Sample stability | Signal intensity of pure buffer | Signal intensity relative to PBS buffer [%] | Signal intensity of rye extract relative to pure buffer [%] | Signal intensity of wheat extract relative to pure buffer [%] |
|---|---|---|---|---|---|
| SDS, polysorbate 20 | mediocre | 4,142 | 81 | 90 | 93 |
| SDS, KCl | stable | 3,695 | 72 | 70 | 69 |
| SDS, MgCl2 | unstable | 4,951 | 96 | 84 | 80 |
| SDS, PAA | unstable | 4,649 | 90 | 101 | 13 |
| polysorbate 20, KCl | mediocre | 2,047 | 40 | 74 | 100 |
| polysorbate 20, MgCl2 | mediocre | 2,898 | 56 | 104 | 108 |
| polysorbate 20, PAA | stable | 3,387 | 66 | 106 | 115 |
| KCl, MgCl2 | mediocre | 3,729 | 73 | 78 | 109 |
| KCl, PAA | stable | 2,693 | 52 | 88 | 83 |
| $MgCl_2$, PAA | stable | 4,517 | 88 | 88 | 84 |

(continued)

| Components (SDS: 0.1%, polysorbate 20: 0.2%, KCl: 100mM, MgCl2: 100 mM, PAA: 1%) | Sample stability | Signal intensity of pure buffer | Signal intensity relative to PBS buffer [%] | Signal intensity of rye extract relative to pure buffer [%] | Signal intensity of wheat extract relative to pure buffer [%] |
|---|---|---|---|---|---|
| SDS, polysorbate 20, KCl | stable | 2,318 | 45 | 92 | 101 |
| SDS, polysorbate 20, MgCl2 | stable | 4,372 | 85 | 85 | 89 |
| SDS, polysorbate 20, PAA | stable | 4,498 | 87 | 93 | 90 |
| SDS, KCl, MgCl2 | stable | 3,832 | 75 | 76 | 94 |
| SDS, KCl, PAA | stable | 4,240 | 82 | 63 | 68 |
| SDS, MgCl$_2$, PAA | mediocre | 5,200 | 101 | 83 | 79 |
| polysorbate 20, KCl, MgCl2 | stable | 1,585 | 31 | 68 | 100 |
| polysorbate 20, KCl, PAA | stable | 4,814 | 94 | 26 | 34 |
| polysorbate 20, MgCl$_2$, PAA | stable | 2,857 | 56 | 104 | 112 |
| KCl, MgCl$_2$, PAA | stable | 2,625 | 51 | 92 | 95 |
| SDS, KCl, MgCl$_2$, PAA | stable | 4,199 | 82 | 57 | 64 |
| polysorbate 20, KCl, MgCl$_2$, PAA | stable | 1,638 | 32 | 61 | 98 |
| SDS, polysorbate 20, MgCl$_2$, PAA | stable | 4,384 | 85 | 85 | 94 |
| SDS, polysorbate 20, KCl, PAA | stable | 2,065 | 40 | 105 | 122 |
| SDS, polysorbate 20, KCl, MgCl$_2$ | stable | 1,870 | 36 | 79 | 102 |
| SDS, polysorbate 20, KCl, MgCl$_2$, PAA | stable | 2,108 | 41 | 73 | 91 |
| None (PBS) | control | 5,141 | 100 | 72 | 86 |

In the next step, we validated our findings with a larger variety of different food samples. Hence, the four best buffers were tested, see Table 3.

Table 3. Buffers for further testing.

| Buffer | Composition |
|---|---|
| 1 | 100 mM MgCl$_2$, 1% PAA |
| 2 | 0.1% SDS, 0.2% polysorbate 20, 100 mM MgCl$_2$ |
| 3 | 0.1% SDS, 0.2% polysorbate 20, 1% PAA |
| 4 | 0.1% SDS, 0.2% polysorbate 20, 100 mM MgCl$_2$, 1% PAA |
| PBS (control) | |

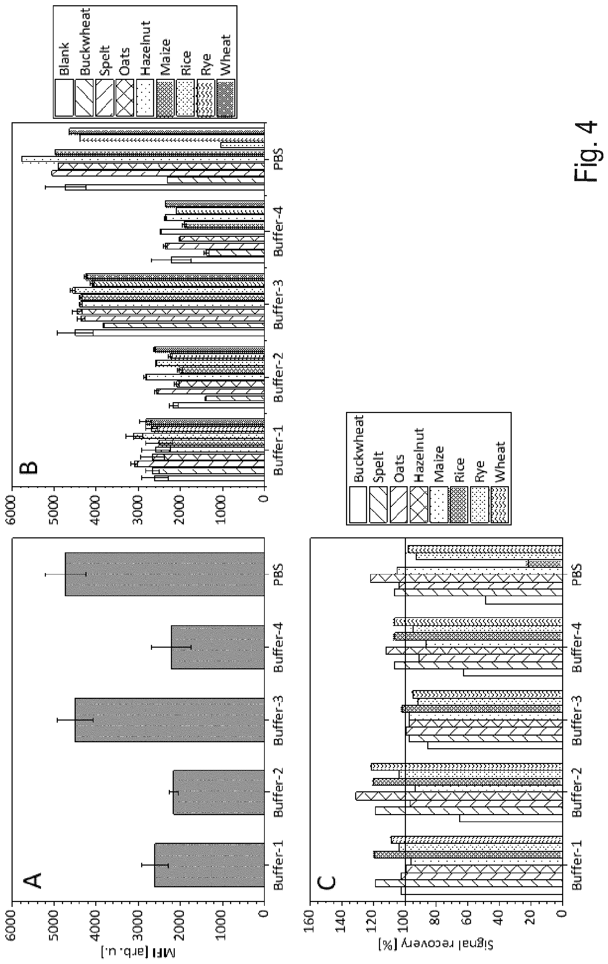

As can be seen in Fig. 4A, Buffer 3 (0.1% SDS, 0.2% polysorbate 20 and 1% PAA) has the highest signal in comparison to the PBS-control. Additionally, recovery rates were the highest in Buffer 3 and spanned from 85-101%. Therefore, Buffer 3 fulfilled the criterium of having a better signal recovery of at least 80%. All other buffers had either a lower signal recovery or a too low signal intensity of the blank.

Further addition of NREs or the addition or replacement of other detergents (sodium deoxycholate, laureth-4, laureth-23, ceteth-2, ceteth-20, and octxinol-9) to the sample buffer did not improve the results any further. Also, substitution of PAA by PVA and/or PVP did not improve the results.

Optimization of the secondary antibody buffer

To boost the assay sensitivity the buffer of the secondary antibodies was optimized. This was further necessary to compensate for the slight signal reduction of approximately 10-15% caused by the developed sample buffer. Here we found that NREs (especially laureth-23, Ceteth-2, Ceteth-20) did enhance the signal intensity. The best results were obtained by using laureth-23 at a concentration of 0.2%. The signal intensity of the SAFIA for FB1, DON and OTA could be increased by 11-23% (Table 4) and, hence, it was further used in the SAFIA.

Table 4. Comparison of signal intensities of the optimized buffer for secondary antibodies

| Buffer sec. Antibody | TRIS + 0.2% Laureth-23 | | | TRIS | | |
|---|---|---|---|---|---|---|
| Sample | Intensity FB1 [arb. U.] | Intensity DON [arb. U.] | Intensity OTA [arb. U.] | Intensity FB1 [arb. U.] | Intensity DON [arb. U.] | Intensity OTA [arb. U.] |
| Blank | 4,705 | 6,442 | 4,086 | 4,014 | 5,229 | 3,582 |
| Wheat | 4,867 | 5,879 | 3,740 | 4,253 | 4,927 | 3,383 |
| Rye | 4,737 | 5,298 | 3,697 | 4,094 | 4,490 | 3,293 |
| | Increase of Signal intensity [%] | | | | | |
| Sample | FB1 | DON | OTA | | | |
| Blank | 17 | 23 | 14 | | | |
| Wheat | 14 | 19 | 11 | | | |
| Rye | 16 | 18 | 12 | | | |

Addition of free analyte to the stopping buffer

To improve the stopping buffer, free analyte was added to the stopping solution. The concentration of each analyte was chosen to yield ca. 90% inhibition of the maximum signal. However, smaller concentrations (e.g., IC50) might be preferable for some binders and must be tested prior application. The final optimum concentrations for AFB1, OTA, DON, FB1 and ZEN-Assays are depicted in Table 5.

Table 5. Preferred concentration range of free analyte in the stopping buffer.

| Analyte | Concentration of free analyte in the stopping buffer [$\mu$g/L] |
|---|---|
| AFB1 | 1-5 |
| OTA | 5-100 |
| DON | 30-100 |
| ZEN | 50-100 |
| FB1 | 30-100 |

For comparison of the effects two calibration curves were recorded: one using the conventional stopping solution without the free analytes and one containing the free analytes. The assay was measured two times, separated by a delay of 2 h between each measurement. Comparison of the buffers can be found in Table 6. Without the addition of free analytes, the signal increases during the two hours by ca. 20% for OTA, FB1, ZEN and AFB1. This means that the concentration might be underestimated by >20% in a sample measurement. However, the signal was stabilized by utilizing the improved stopping buffer which contained free analytes. The relative signal deviation was reduced by a factor of around 10 for AFB1, ZEN and OTA and improved for FB1 (factor 4) and DON (factor 1.4). Additionally, this had a significant improvement on the recovery rates. The recovery rates of spiked sample extracts (buckwheat, spelt, oats, rice, rye, almond, hazelnut, and wheat) at a level of 5 $\mu$g/L for ZEN improved from 72% to 107% (mean recovery, see Figure 5). The precision was improved as well. The mean intra-assay coefficient of variation (CV) with the stopping buffer containing free analyte was 2.9%, whereas the mean intra-assay CV using the stopping buffer without free analyte was 7.1%.

Table 6. Comparison of the stopping buffer with and without free analyte. Compared are the A-Values (upper asymptotes) of the calibration curves.

| Analyte | Stopping buffer | A-Value Measurement 1 | A-Value Measurement 2 | Abs. difference (M2-M1) | Relative difference [%] |
|---|---|---|---|---|---|
| OTA | Without free analyte | 2,365 | 2,826 | 460 | 19.5 |
| | Containing free analyte | 1,878 | 1,842 | -36 | -1.92 |
| DON | Without free analyte | 3,877 | 3,636 | -241 | -6.21 |
| | Containing free analyte | 3,059 | 2,919 | -140 | -4.58 |
| FB1 | Without free analyte | 4,503 | 5,105 | 602 | 13.4 |
| | Containing free analyte | 4,116 | 3,971 | -145 | -3.52 |
| ZEN | Without free analyte | 2,315 | 2,737 | 422 | 18.2 |
| | Containing free analyte | 1,876 | 1,897 | 21 | 1.12 |
| AFB1 | Without free analyte | 1,530 | 1,826 | 296 | 19.4 |
| | Containing free analyte | 892 | 866 | -26 | -2.91 |

Analysis of spiked food-extracts

Eventually, we proved that our buffer system allowed for highly accurate sample quantification by the analysis of 8 different food matrices (buckwheat, spelt, oats, rice, rye, almond, hazelnut, and wheat) from commercial sources. All samples were extracted with 70% MeOH, which is a standard method for mycotoxin analysis. Then, they were diluted in the developed sample buffer and aliquoted. The first aliquot was analysed directly with SAFIA, the second was spiked with the four mycotoxins of following concentrations (Table 7). All samples were spiked in the same manner.

Table 7. Spiking levels in reference samples.

| Analyte | Spiked concentration [$\mu$g/L] |
|---|---|
| OTA | 1.0 |
| DON | 100 |
| ZEN | 5.0 |
| FB1 | 10 |

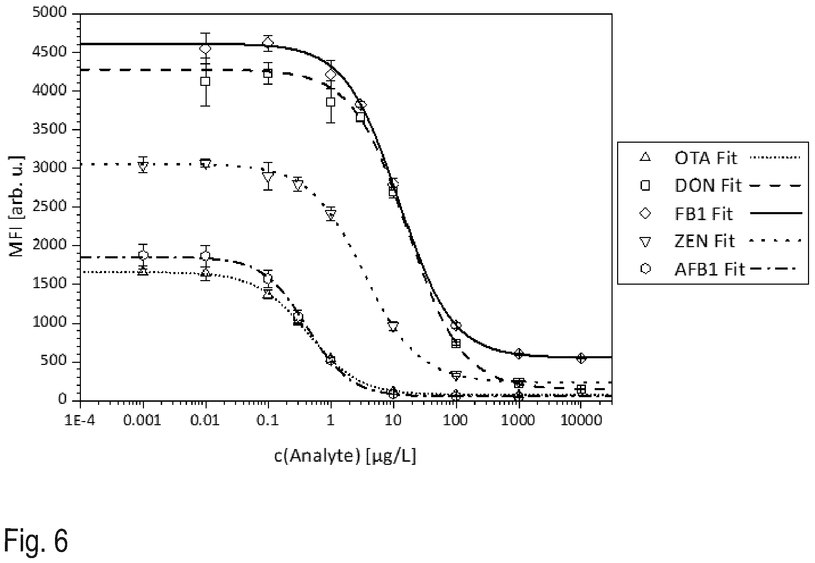

A standard curve was obtained in triplicate measurements, see Figure 6. Fit Parameters and Quality Parameters are given in Table 8 below. As an estimate for limits of detection (LOD) we have chosen the concentration giving 20% inhibition (IC20). Hence, the LODs were 0.137 $\mu$g/L for AFB1, 0.119 $\mu$g/L for OTA, 4,11 $\mu$g/L for DON, 3.12 $\mu$g/L for FB1 and 0.837 $\mu$g/L for ZEN. All non-spiked samples showed a concentration below the LODs and where therefore successfully analysed as correct-negative, see Table 8.

Table 8. Obtained parameters of the calibration curves for OTA, DON, FB1, ZEN and AFB1.

| Parameter | OTA | DON | FB1 | ZEN | AFB1 |
|---|---|---|---|---|---|
| A1 [arb. U.] | 1,658 | 4,276 | 4,614 | 3,056 | 1,853 |
| A2 [arb. U.] | 75 | 147 | 552 | 238 | 54 |
| IC50 [$\mu$g/L] | 0.432 | 16.6 | 12.2 | 3.38 | 0.414 |
| IC20 [$\mu$g/L] | 0.119 | 4.11 | 3.12 | 0.837 | 0.137 |
| IC80 [$\mu$g/L] | 1.56 | 67.2 | 47.5 | 13.7 | 1.25 |
| P | 1.077 | 0.992 | 1.018 | 0.992 | 1.250 |
| Chi Square | 0.107 | 0.310 | 0.176 | 0.120 | 0.384 |
| R Square | 0.9999 | 0.9998 | 0.9999 | 0.9999 | 0.9983 |
| Dynamic Range [arb.U.] | 1,583 | 4,129 | 4,061 | 2,817 | 1,798 |
| relative Dynamic Range [%] | 95.5 | 96.6 | 88.0 | 92.2 | 97.1 |

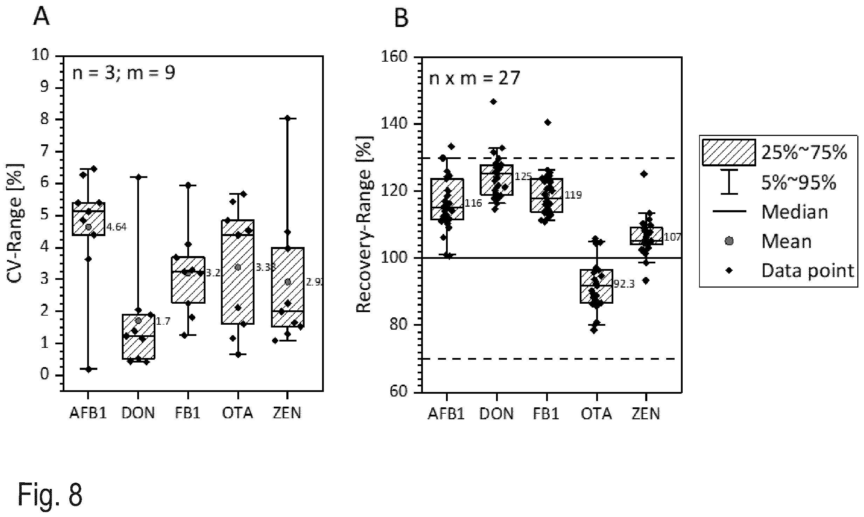

The analysis of the spiked extracts was done with a very good accuracy. The intra-assay precision of the assay was very high, mean coefficient of variations (CV) was below 5%. The recovery rates were very high as well and in an optimum range between 70-130% (ideal: 100%), see Figure 8.

The invention can alternatively be described by the aspects listed below.

1. Dilution buffer for extracted food and feed samples composed of a detergent with high HLB (8-13) and a detergent with low HLB (14-20) and a polyanionic polymeric species.

2. Dilution buffer with SDS in the concentration range of 0.05-0.15% (m/m) (preferred: 0.1% (m/m)) and polysorbate 20 in the concentration range of 0.15-0.3% (m/m) (preferred: 0.2% (m/m)) and sodium salt of polyacrylic acid (with an average molecular weight of 2100 Da) in the concentration range between 0.5-3% (m/m) with a preferred concentration of 0.1% (m/m).

3. Stopping buffer for SAFIA that contains free analytes in combination with a crosslinker (glutaraldehyde), MeOH and SDS.

4. In combination with 1 or 2 a buffer for secondary antibodies containing a polyglycol ethers of fatty acids in the concentration range of 0.1-0.3% (m/m) (preferred range: 0.2% (m/m))

5. In combination with 1 or 2 and 3 a buffer for secondary antibodies containing laureth-23 to enhance the luminescence of secondary antibodies.

6. A kit containing compounds 1 or 2 for preparation of samples for immunoassays.

7. A SAFIA kit containing compounds of 1 or 2 and 3 and 4 or 5 and all necessary reagents for executing a particle-based immunoassay, i.e., microparticles, primary antibodies, luminophore labelled secondary antibodies and/or other binder and/or luminescent reporter molecules.

8. A SAFIA kit containing compounds of 1 or 2 and 3 and 4 or 5 and all necessary reagents for executing a particle-based immunoassay, i.e., microparticles, primary antibodies, luminophore labelled secondary antibodies and/or other binder and/or luminescent reporter molecules for detection of mycotoxins (preferred: Ochratoxin A, Deoxynivalenol, Zearalenone, Fumonisins (B1 and/orB2) and Aflatoxins (B1 and or B2 and/or G1 and/or G2 and/or M1)) and ergot alkaloids (ergocornine/ergocorninine, ergocristine/ergocristinine, ergocryptine/ergocryptinine ($\alpha$- and $\beta$-form), ergometrine/ergometrinine, ergosine/ergosinine, ergotamine/ergotaminine) and T2and/or HT2 toxin in food, feed or other sample material.

REFERENCES

1. EP 3 771 487 A1.

2. Sarma, D.; Carl, P.; Climent, E.; Schneider, R. J. and Rurack, K. Multifunctional Polystyrene Core/Silica Shell Microparticles with Antifouling Properties for Bead-based Multiplexed and Quantitative Analysis. ACS Appl. Mater. Interfaces, 2019. 11(1): 1321-1334.

3. Carl, P.; Sarma, D.; Gregório, B. J. R.; Hoffmann, K.; Lehmann, A.; Rurack, K. and Schneider, R. J. Wash-Free Multiplexed Mix-and-Read Suspension Array Fluorescence Immunoassay for Anthropogenic Markers in Wastewater.

Anal. Chem., 2019. 91(20): 12988-12996.

4. Thouvenot, D. and Morfin, R. F. Radioimmunoassay for Zearalenone and Zearalanol in Human Serum: Production, Properties, and Use of Porcine Antibodies. Appl. Environ. Microbiol., 1983. 45: 16-23.

5. Hastings, K. L.; Hagler, W. M. J.; Harris, T. D.; Voyksner, R. D. and Dean, J. H. Production and Characterization of Aflatoxin B2 Oximinoacetate. J. Agric. Food Chem., 1989. 37(2): 393-400.

6. Casale, W. L.; Pestka, J. J. and Hart, L. P. Enzyme-Linked Immunosorbent-Assay Employing Monoclonal-Antibody Specific for Deoxynivalenol (Vomitoxin) and Several Analogs. J. Agric. Food Chem., 1988. 36(3): 663-668.

The present invention has been explained with reference to various illustrative embodiments and examples. These embodiments and examples are not intended to restrict the scope of the invention, which is defined by the claims and their equivalents. As is apparent to one skilled in the art, the embodiments described herein can be implemented in various ways without departing from the scope of what is invented. Various features, aspects, and functions described in the embodiments can be combined with other embodiments.

## Claims

1. A buffer system for detecting an analyte using a suspension array fluorescence immunoassay (SAFIA) comprising:

   a) an extract dilution buffer for diluting an extract of a food or a feed sample to be analysed in the SAFIA, the extract dilution buffer comprising:

   - a first detergent having a high hydrophilic-lipophilic balance (HLB) ,
   - a second detergent having a low HLB, and
   - a polyanionic species,

   wherein the high HLB is selected from a range of HLB-values of 8 through 13, and the low HLB is selected from the range of HLB-values of 14 through 20;
   b) a stopping buffer for stabilizing a read-out signal of the SAFIA by preventing unbound antibodies form reacting with a corresponding epitope, the stopping buffer comprising:

   - the analyte,
   - a protein crosslinker,
   - MeOH and
   - SDS,

   wherein the protein crosslinker is selected from formaldehyde or a linear and/or branched alkane or aromatic dialdehyde selected from: glyoxal, succinic aldehyde, glutaraldehyde, adipaldehyde, benzene dicarbaldehyde, and genipin; and
   c) a secondary antibody dilution buffer for diluting a secondary antibody comprising the extract dilution buffer and a polyglycol ether of a fatty acid,

   wherein the secondary antibody is directed towards a primary antibody and comprises a luminescence label, and
   wherein the first antibody is directed towards the analyte and/or a hapten fixed at a particle's surface.

2. The buffer system according to claim 1, wherein a concentration of the polyglycol ether of the fatty acid is selected from a value of 0.1 - 0.3% (m/m), preferably comprising 0.2% (m/m).

3. The buffer system according to claim 1 or 2, wherein the first detergent having the high HLB is selected from SDS, and a concentration of SDS is selected from a value of 0.05 - 0.15% (m/m), preferably comprising 0.1% (m/m), and wherein the second detergent having the low HLB is selected from polysorbate 20, wherein a concentration range of polysorbate 20 is selected from a value of 0.15 - 0.3% (m/m), preferably comprising 0.2% (m/m).

4. The buffer system according to any of claims 1-3, wherein the polyanionic species is selected from a polyanionic polymer, wherein pendant groups of the polyanionic polymer are selected from: hydroxyl, carboxyl, sulfonate, sulfate, sulfite, phosphate, and phosphonate groups and their combinations; and a backbone of the polyanionic polymer is selected from: a polyvinyl, a polystyrene, a polyacrylic, and a polyglycoside, wherein the polyanionic polymer comprises: a polystyrene sulfonate, a polyvinyl sulfonate, an alginic acid, a polyacrylamidomethylpropane sulfonic acid, a polyvinyl acetic acid, a polyvinyl phosphonic acid, a polyvinyl sulfonic acid, a carboxymethyl cellulose, a carrageenan, a polyaspartic acid, a polyglutamic acid, a polyphosphoric acid, a polysaccharide, a pectin, a polyacrylic acid and a heparin, particularly a sodium salt of the polyacrylic acid, wherein the polyacrylic acid has an average molecular weight of 1800 through 2200 Da, preferably comprising 2100 Da, and wherein a concentration of the sodium salt of the polyacrylic acid is selected from a value of 0.5 - 3% (m/m), preferably comprising 0.1% (m/m).

5. The buffer system according to any of claims 1-4, wherein the stopping buffer is configured to stabilize a bond between a secondary antibody or a primary antibody and an entity which is specifically recognized by said primary or secondary antibody, wherein said entity typically comprises the primary antibody which has already bound either the corresponding hapten fixed at a particle's surface or the corresponding analyte dissolved in the sample extract.

6. The buffer system according to any of claims 1-5, wherein the secondary antibody dilution buffer further comprises a laureth-23 concentration range of 0.1 -0.3%, wherein the laureth-23 enhances a luminescence of the luminescence label.

7. The buffer system according to any of claims 1-6, wherein an aqueous phosphate buffered saline (PBS) is used as a solvent for the buffer system, wherein the PBS typically comprises 100-300 mg/L potassium chloride; 100-300 mg/L sodium or potassium dihydrogen phosphate; 900 -1500 mg/L di-sodium or di-potassium hydrogen phosphate (anhydrous); and 6000-10000 mg/L sodium chloride.

8. The buffer system according to any of claims 1-6, wherein an aqueous TRIS buffered saline (TBS) is used as a solvent for the buffer system, wherein the TBS typically comprises 800-1500 mg/L Tris(hydroxymethyl)aminomethane and 6000-10000 mg/L sodium chloride and optionally 100-300 mg/L potassium chloride.

9. The buffer system according to any of claims 1-6, wherein an aqueous borate buffered saline (BBS) is used as a solvent for the buffer system, wherein the BBS typically comprises 1900-12,000 mg/L sodium borate decahydrate (borax) and 6000-10000 mg/L sodium chloride and optionally 100-300 mg/L potassium chloride.

10. A kit for a SAFIA comprising the buffer system according to any of claims 1-9.

11. The kit according to claim 10, further comprising:

  - at least two different lots of core/shell microparticles carrying at their surface different haptens or analytes, at least two primary antibodies, each of the two antibodies specifically directed to another one of the different haptens or analytes.

12. The kit according to claim 10 or 11, wherein any of the primary antibodies is directed to another mycotoxin, selected from: an Ochratoxin A, a Deoxynivalenol, a Zearalenone, a Fumonisin (preferably Fumonisin B1 or Fumonisin B2), an Aflatoxin (preferably Aflatoxin B1, Aflatoxin B2, Aflatoxin G1, Aflatoxin G2 or Aflatoxin M1), an ergot alkaloid (preferably ergocornine/ergocorninine, ergocristine/ergocristinine, ergocryptine/ergocryptinine ($\alpha$- and $\beta$-forms), ergometrine/ergometrinine, ergosine/ergosinine or ergotamine/ergotaminine); T2 toxin; and HT2 toxin.

13. The kit according to claim 12, further comprising:

  - a secondary antibody which is labelled with a luminophore and directed towards at least one of the primary antibodies and/or
  - another binder molecule carrying a luminescent reporter.

14. Using the buffer system according to any of claims 1-9 or the kit according to any of claims 10-13 for determining a concentration of a contaminant in an extract of a sample, wherein the contaminant is selected from a pesticide, an antibiotic, and an allergenic compound, and wherein the sample is selected from a food, a feed, and an agricultural product.

15. Using the buffer system according to claim 14, wherein the contaminant is a mycotoxin.

Fig. 1

Fig. 2

Fig. 3

Fig. 4

Fig. 5

Fig. 6

Fig. 7

Fig. 8

Europäisches
Patentamt
European
Patent Office
Office européen
des brevets

# EUROPEAN SEARCH REPORT

Application Number

EP 22 15 3689

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | EP 3 771 487 A1 (BUNDESREPUBLIK DEUTSCHLAND [DE]) 3 February 2021 (2021-02-03) * the whole document * * claims 1-13 * | 1-15 | INV. G01N33/53 G01N33/543 G01N15/14 |
| A,D | CARL P. ET AL.: "Wash-free multiplexed mix-and-read suspension array fluorescence immunoassay for anthropogenic markers in wastewater", ANAL. CHEM., vol. 91, no. 20, 19 September 2019 (2019-09-19), pages 12988-12996, XP055760480, * the whole document * * figure 1 * | 1-15 | |
| A | JIANWEN Q. ET AL.: "Multiplex flow cytometric immunoassays for high-throughput screening of multiple mycotoxin residues in milk", FOOD ANAL. METHODS, vol. 12, no. 4, 4 January 2019 (2019-01-04), pages 877-886, XP036743008, * the whole document * * figure 2 * | 1-15 | |
| A | SU P. ET AL.: "Simultaneous detection of five antibiotics in milk by high-throughput suspension array technology", TALANTA, vol. 85, no. 2, 19 May 2011 (2011-05-19), pages 1160-1165, XP028097075, * the whole document * | 1-15 | |

TECHNICAL FIELDS
SEARCHED (IPC)

G01N

-/--

The present search report has been drawn up for all claims

| Place of search | Date of completion of the search | Examiner |
|---|---|---|
| Munich | 7 July 2022 | Giry, Murielle |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 1 of 2

**EUROPEAN SEARCH REPORT**

Application Number

EP 22 15 3689

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DENG G. ET AL.: "High sensitive immunoassay for multiplex mycotoxin detection with photonic crystal microsphere suspension array", ANAL. CHEM., vol. 85, no. 5, 25 January 2013 (2013-01-25), pages 2833-2840, XP055760657, * the whole document * | 1-15 | |

TECHNICAL FIELDS
SEARCHED        (IPC)

The present search report has been drawn up for all claims

| Place of search | Date of completion of the search | Examiner |
|---|---|---|
| Munich | 7 July 2022 | Giry, Murielle |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 15 3689

This annex lists the patent family members relating to the patent documents cited in  the above-mentioned European search report.
The members are as contained in the European Patent Office EDP file on
The European Patent Office is in no way liable for these particulars which are merely given for the purpose of information.

07-07-2022

| Patent document cited in search report | | Publication date | Patent family member(s) | | Publication date |
|---|---|---|---|---|---|
| EP 3771487 | A1 | 03-02-2021 | DE 102019120455 | A1 | 04-02-2021 |
| | | | EP 3771487 | A1 | 03-02-2021 |

For more details about this annex : see Official Journal of the European Patent Office, No. 12/82

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3771487 A1

**Non-patent literature cited in the description**

- **SARMA, D. ; CARL, P. ; CLIMENT, E. ; SCHNEI-DER, R. J. ; RURACK, K.** Multifunctional Polystyrene Core/Silica Shell Microparticles with Antifouling Properties for Bead-based Multiplexed and Quantitative Analysis. *ACS Appl. Mater. Interfaces,* 2019, vol. 11 (1), 1321-1334
- **CARL, P. ; SARMA, D. ; GREGÓRIO, B. J. R. ; HOFFMANN, K. ; LEHMANN, A. ; RURACK, K. ; SCHNEIDER, R. J.** Wash-Free Multiplexed Mix-and-Read Suspension Array Fluorescence Immunoassay for Anthropogenic Markers in Wastewater. *Anal. Chem.,* 2019, vol. 91 (20), 12988-12996

- **THOUVENOT, D. ; MORFIN, R. F.** Radioimmunoassay for Zearalenone and Zearalanol in Human Serum: Production, Properties, and Use of Porcine Antibodies. *Appl. Environ. Microbiol.,* 1983, vol. 45, 16-23
- **HASTINGS, K. L. ; HAGLER, W. M. J. ; HARRIS, T. D. ; VOYKSNER, R. D. ; DEAN, J. H.** Production and Characterization of Aflatoxin B2 Oximinoacetate. *J. Agric. Food Chem.,* 1989, vol. 37 (2), 393-400
- **CASALE, W. L. ; PESTKA, J. J. ; HART, L. P.** Enzyme-Linked Immunosorbent-Assay Employing Monoclonal-Antibody Specific for Deoxynivalenol (Vomitoxin) and Several Analogs. *J. Agric. Food Chem.,* 1988, vol. 36 (3), 663-668